# EUROPEAN PATENT APPLICATION

(11) **EP 1 733 760 A1**
(43) Date of publication of application: **20.12.2006**
(21) Application number: 05005441.0
(22) Date of filing: 12.03.2005
(51) Int. Cl.: A61Q 5/10

(54) **Colourants for keratin fibres comprising certain cationic cellulose ether derivatives**

(71) Applicant: Wella Aktiengesellschaft, 64274 Darmstadt (DE)
(72) Inventor: Schmenger, Jürgen, 64331 Weiterstadt (DE); Kujawa, Jolanthe, 64289 Darmstadt (DE); Otto, Kathrin, 64380 Rossdorf (DE)

(57) **Abstract**

The present application relates to an agent for colouring keratin fibres based on oxidation dye precursors and/or direct dyes, which is characterized in that it comprises, in a suitable cosmetic carrier, at least one cellulose ether with 4,000 to 10,000 anhydroglucose units, which is substituted by (a) per mole of anhydroglucose unit on average 0.0003 to 0.08 mol of a nonionic or cationic hydrophobic substituent containing an alkyl group or arylalkyl group having 8 to 24 carbon atoms and (b) a cationic substituent of the formula (I),

[R¹R²R³R⁴N⁺] (A^{z-})_{1/z} (I)

where **R1, R2** and **R3**, independently of one another, are a methyl or ethyl group,
**R4** is a -CH2-CH2 group or a -CH2-CHOH-CH2 group,
**A^{z-}** is an anion and **z** is 1, 2 or 3.

## Description

The invention relates to agents for colouring keratin fibres, in particular human hair, with a content of direct and/or oxidative dyes and certain cationic cellulose ether derivatives.

Colouring preparations are usually in the form of aqueous - preferably thickened - solutions or emulsions and, besides dyes, comprise, for example, fatty alcohols and/or other oil components, emulsifiers and surfactants, and, if appropriate, alcohols. Oxidation colorants generally consist of two components, (i) the dye carrier mass comprising the dyes and (ii) the oxidizing agent preparation, which are mixed together shortly before use and then applied to the hair to be coloured. A care effect after rinsing out the oxidation colorant is desired. A greater or lesser care effect results depending on the care raw materials used, but this can in no way replace the use of a conditioner to be applied subsequently.

Upon mixing, the viscosity and mixing ratio of the two components determine how high or low the viscosity is. Good adhesion of the colorant is achieved here in particular through a relatively high viscosity of the colorant. In addition, the hair stylist often requires relatively high viscosities for his work, for example for special tress or foil techniques, and in order to be able to perform targeted work using the colouring brush or the accentuating brush.

There was therefore a great need for a cost-effective thickening of the dye carrier mass which ensures a good miscibility of the dye carrier mass with the oxidizing agent and produces colorants with good adhesion properties and colouring properties and which also has improved care properties compared with compositions according to the prior art.

The use of cationic hydroxyethylcelluloses in hair colorants is sufficiently known. However, the agents described therein are not satisfactory in every respect with regard to their viscosity and their adhesion on the hair, the care properties, and the colouring properties. The cellulose derivatives used according to the prior art increase the viscosity of the oxidation colorants only insignificantly and the care effect is unsatisfactory.

In this respect, it has now been found that the abovementioned disadvantages of the cellulose derivatives used hitherto can be overcome through the use of certain cationic cellulose ether derivatives in an excellent way, and both an excellent care and also a very good increase in viscosity can be achieved.

The present invention therefore provides an agent for colouring keratin fibres, in particular human hair, based on oxidation dye precursors and/or direct dyes, which is characterized in that it comprises, in a suitable cosmetic carrier, at least one cellulose ether with 4,000 to 10,000 anhydroglucose units, which is substituted by (a) per mole of anhydroglucose unit on average 0.0003 to 0.08 mol of a nonionic or cationic hydrophobic substituent containing an alkyl group or arylalkyl group having 8 to 24 carbon atoms and (b) a cationic substituent of the formula (I),

[R¹R²R³R⁴N⁺] (A^{z-})_{1/z} (I)

where **R1, R2** and **R3,** independently of one another, are a methyl or ethyl group,
**R4** is a -CH2-CH2 group or a -CH2-CHOH-CH2 group,
**A^{z-}** is an anion, for example a phosphate anion, nitrate anion, sulphate anion or halogen anion and in particular a chloride anion, and
**z** is 1, 2 or 3.

The hydrophobic substituent (a) used is preferably a compound of the formula (II)

[R⁵R⁶R⁷R⁸N⁺](A^{z-})_{1/z} (II)

where **R5 and R6,** independently of one another, are a methyl group or ethyl group,
**R7** is a -CH2-CH2 group or a -CH2-CHOH-CH2 group,
**R8** is an alkyl or arylalkyl group having 8 to 24 carbon atoms
**A^{z-}** is an anion, for example a phosphate anion, nitrate anion, sulphate anion or halogen anion and in particular a chloride anion, and
zis1,2or3.

Particular preference is given to cellulose ethers in which, in the formula (II), the following apply: R5 = methyl and in particular R5 = R6 = methyl, where R7 is preferably a -CH2-CHOH-CH2 group and R8 is an alkyl group having 8 to 24 carbon atoms, in particular 10 to 18 carbon atoms. Preference is likewise given to hydrophobic substituents (a) in which R8 is a glycidyl ether, for example nonylphenyl glycidyl ether or dodecylphenyl glycidyl ether, alpha-olefin epoxides, for example 1,2-epoxyhexadecanes and chlorohydrins thereof, or alkyl halides, for example dodecyl bromide. It is likewise possible to use mixtures of the abovementioned hydrophobic substituents (a).

Particular preference is given to cellulose ethers in which, in the formula (I), the following apply: R1 = methyl, in particular R1 = R2 = R3 = methyl and R4 = -CH2-CHOH-CH2-, where the degree of substitution of the cationic substituent (b) is on average about 0.002 to 0.9 mol, preferably about 0.05 to 0.8 mol, and in particular about 0.1 to 0.6 mol.

The cellulose ethers according to the invention preferably have a viscosity of from 1,500 to 350,000 mPas, particular preference being given to a viscosity of from 2,000 to 150,000 mPas and in particular 50,000 to 90,000 mPas (measured in each case as 2% strength aqueous solution at 25 °C using a Brookfield viscometer).

Particularly suitable cellulose ethers are those in which the hydrophobic substituent (a) is a [-O-(CH₂CH₂O)ₓ-(CH₂CHOHCH-N(CH₃)₂(C₁₂H₂₅)⁺Cl⁻)_{y'}-H] group and the cationic substituent (b) is a [-O-(CH₂CH₂O)ₓ-(CH₂CHOHCH-N(CH₃)₃⁺Cl⁻)_{y}-H] group. Such polymers are sold by Dow Corning under the trade name Softcat® Polymer SL-5, Softcat® Polymer SL-30, Softcat® Polymer SL-60 or Softcat® Polymer SL-100. The abovementioned cationic cellulose ethers and the preparation thereof are described in WO 2005/000903 - to which reference is expressly made here.

The cationic celluloses are present in the colorant according to the invention (in each case based on the active substance) preferably in an amount of from about 0.01 to 20% by weight, in particular from about 0.1 to 5% by weight.

The colorant according to the invention preferably comprises oxidation dye precursors, with which the coloration is produced under the action of oxidizing agents, such as, for example, hydrogen peroxide, or in the presence of atmospheric oxygen.

Suitable oxidation dye precursors which may be specified are, for example, the following developer substances and coupler substances and self-coupling compounds:
(i) Developer substances: 1,4-diaminobenzene (p-phenylenediamine), 1,4-diamino-2-methylbenzene (p-tolylenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)-amino]aniline, 4-[di(2-hydroxyethyl)amino]aniline, 4-[di(2-hydroxyethyl)-amino]-2-methylaniline, 4-[(2-methoxyethyl)amino]aniline, 4-[(3-hydroxypropyl)amino]aniline, 4-[(2,3-dihydroxypropyl)amino]aniline, 1,4-diamino-2-(2-hydroxyethyl)benzene, 1,4-diamino-2-(1-methylethyl)benzene, 1,3-bis[(4-aminophenyl)(2-hydroxyethyl)amino]-2-propanol, 1,4-bis[(4-aminophenyl)amino]butane, 1,8-bis(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol, alone or in a mixture with one another.
(ii) Coupler substances: N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)-pyridine, 2,6-diamino-3,5-dimethoxypyridine, 3,5-diamino-2,6-dimethoxy-pyridine, 1,3-diaminobenzene, 2,4-diamino-1-(2-hydroxyethoxy)benzene, 1,3-diamino-4-(2,3-dihydroxypropoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diamino-phenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 3-amino-2,6-dimethylphenol, 2-methyl-5-(β-hydroxyethylamino)phenol, 5-amino-2-ethylphenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]-phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)-amino]-2-methylphenol, 3-[(2-hydroxyethyl)amino]-2-methylphenol, 2-amino-3-hydroxypyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole, 2,3-indolenedione, alone or in a mixture with one another.
(iii) Self-coupling compounds: 2-amino-5-methylphenol, 2-amino-6-methylphenol, 2-amino-5-ethoxyphenol or 2-propylamino-5-aminopyridine.

The total amount of the oxidation dye precursors present in the agent according to the invention is up to about 12 per cent by weight, in particular about 0.2 to 6 per cent by weight.

To achieve certain colour nuances, customary natural and/or synthetic direct dyes, for example so-called plant dyes, such as henna or indigo, triphenylmethane dyes, aromatic nitro dyes, azo dyes, quinone dyes, cationic or anionic dyes, may additionally also be present in the colorant.

Examples of suitable synthetic dyes are: 1,4-bis[(2-hydroxyethyl)amino]-2-nitrobenzene, 1-(2-hydroxyethyl)amino-2-nitro-4-[di(2-hydroxyethyl)-amino]benzene (HC Blue No. 2), 1-amino-3-methyl-4-[(2-hydroxyethyl)-amino]-6-nitrobenzene (HC Violet No. 1), 4-[ethyl-(2-hydroxyethyl)amino]-1-[(2-hydroxyethyl)amino]-2-nitrobenzene hydrochloride (HC Blue No. 12), 4-[di(2-hydroxyethyl)amino]-1-[(2-methoxyethyl)amino]-2-nitrobenzene (HC Blue No. 11), 1-[(2,3-dihydroxypropyl)amino]-4-[methyl-(2-hydroxyethyl)amino]-2-nitrobenzene (HC Blue No. 10), 1-[(2,3-dihydroxypropyl)amino]-4-[ethyl-(2-hydroxyethyl)amino]-2-nitrobenzene hydrochloride (HC Blue No. 9), 1-(3-hydroxypropylamino)-4-[di(2-hydroxyethyl)amino]-2-nitrobenzene (HC Violet No. 2), 1-methylamino-4-[methyl-(2,3-dihydroxypropyl)amino]-2-nitrobenzene (HC Blue No. 6), 2-((4-amino-2-nitrophenyl)amino)-5-dimethylaminobenzoic acid (HC Blue No. 13), 1-amino-4-[(2-hydroxyethyl)amino]-2-nitrobenzene (HC Red No. 7), 2-amino-4,6-dinitrophenol, 4-amino-2-nitrodiphenylamine (HC Red No. 1), 1-amino-4-[di(2-hydroxyethyl)amino]-2-nitrobenzene hydrochloride (HC Red No. 13), 1-amino-5-chloro-4-[(2-hydroxyethyl)amino]-2-nitrobenzene, 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-[(2-aminoethyl)amino]-4-(2-hydroxyethoxy)-2-nitrobenzene (HC Orange No. 2), 4-(2,3-dihydroxypropoxy)-1-[(2-hydroxyethyl)amino]-2-nitrobenzene, (HC Orange No. 3), 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-[(2-hydroxyethyl)amino]-4,6-dinitrophenol, 4-ethylamino-3-nitrobenzoic acid, 2-[(4-amino-2-nitrophenyl)amino]benzoic acid, 2-chloro-6-ethylamino-4-nitrophenol, 2-amino-6-chloro-4-nitrophenol, 4-[(3-hydroxypropyl)amino]-3-nitrophenol, 2,5-diamino-6-nitropyridine, 1,2,3,4-tetrahydro-6-nitroquinoxaline, 7-amino-3,4-dihydro-6-nitro-2H-1,4-benzoxazine (HC Red No. 14), 1-amino-2-[(2-hydroxyethyl)amino]-5-nitrobenzene (HC Yellow No. 5), 1-(2-hydroxyethoxy)-2-[(2-hydroxyethyl)amino]-5-nitrobenzene (HC Yellow No. 4), 1-[(2-hydroxyethyl)amino]-2-nitrobenzene (HC Yellow No. 2), 2-[(2-hydroxyethyl)amino]-1-methoxy-5-nitrobenzene, 2-amino-3-nitrophenol, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC Yellow No. 11), 3-[(2-aminoethyl)amino]-1-methoxy-4-nitrobenzene hydrochloride, (HC Yellow No.9), 1-[(2-ureidoethyl)amino]-4-nitrobenzene, 4-[(2,3-dihydroxypropyl)amino]-3-nitro-1-trifluoromethylbenzene (HC Yellow No. 6), 1-chloro-2,4-bis[(2-hydroxyethyl)amino]-5-nitrobenzene (HC Yellow No. 10), 4-[(2-hydroxyethyl)amino]-3-nitro-1-methylbenzene, 1-chloro-4-[(2-hydroxyethyl)amino]-3-nitrobenzene (HC Yellow No. 12), 4-[(2-hydroxyethyl)amino]-3-nitro-1-trifluoromethylbenzene (HC Yellow No. 13), 4-[(2-hydroxyethyl)amino]-3-nitrobenzonitrile (HC Yellow No. 14), 4-[(2-hydroxyethyl)amino]-3-nitrobenzamide (HC Yellow No. 15), 1,4-di[(2,3-dihydroxypropyl)amino]-9,10-anthraquinone, 1-[(2-hydroxyethyl)amino]-4-methylamino-9,10-anthraquinone (CI61505, Disperse Blue No. 3), 2-[(2-aminoethyl)amino]-9,10-anthraquinone (HC Orange No. 5), 1-hydroxy-4-[(4-methyl-2-sulphophenyl)amino]-9,10-anthraquinone, 1-[(3-aminopropyl)amino]-4-methylamino-9,10-anthraquinone (HC Blue No. 8), 1-[(3-aminopropyl)aminoJ-9,10-anthraquinone (HC Red No. 8), 1,4-diamino-2-methoxy-9,10-anthraquinone (CI62015, Disperse Red No. 11, Solvent Violet No. 26), 1,4-dihydroxy-5,8-bis[(2-hydroxyethyl)amino]-9,10-anthraquinone (CI62500, Disperse Blue No. 7, Solvent Blue No. 69), 9-(dimethylamino)benzo[a]phenoxazin-7-ium chloride (CI51175; Basic Blue No. 6), 1-((4-amino-3,5-dimethylphenyl)(2,6-dichlorophenyl)-methylene)-3,5-dimethyl-4-imino-2,5-cyclohexadiene with phosphoric acid (1:1) (Basic Blue 77), di[4-(diethylamino)phenyl][4-(ethylamino)naphthyl]-carbenium chloride (CI42595; Basic Blue No. 7), 3,7-di(dimethylamino)-phenothiazin-5-ium chloride (CI52015; Basic Blue No. 9), di[4-(dimethylamino)phenyl][4-(phenylamino)-naphthyl]carbenium chloride (CI44045; Basic Blue No. 26), 2-[(4-(ethyl(2-hydroxyethyl)amino)-phenyl)azo]-6-methoxy-3-methylbenzothiazolium methylsulphate (CI11154; Basic Blue No. 41), 8-amino-2-bromo-5-hydroxy-4-imino-6-[(3-(trimethylammonio)phenyl)amino]-1 (4H)-naphthalenone chloride (CI56059; Basic Blue No. 99), bis[4-(dimethylamino)phenyl][4-(methyl-amino)phenyl]carbenium chloride (CI42535; Basic Violet No. 1), tris[4-(dimethylamino)phenyl]carbenium chloride (CI42555; Basic Violet No. 3), 2-[3,6-(diethylamino)dibenzopyranium-9-yl]benzoyl chloride (CI45170; Basic Violet No. 10), di(4-aminophenyl)(4-amino-3-methylphenyl)carbenium chloride (CI42510; Basic Violet No. 14), 1,3-bis[(2,4-diamino-5-methylphenyl)azo]-3-methylbenzene (CI21010; Basic Brown No. 4), 1-[(4-aminophenyl)azo]-7-(trimethylammonio)-2-naphthol chloride (CI12250; Basic Brown No. 16), 1-[(4-amino-2-nitrophenyl)azo]-7-(trimethylammonio)-2-naphthol chloride (Basic Brown No. 17), 1-[(4-amino-3-nitrophenyl)azo]-7-(trimethylammonio)-2-naphthol chloride (CI12251; Basic Brown No. 17), 3,7-diamino-2,8-dimethyl-5-phenylphenazinium chloride (CI50240; Basic Red No. 2), 1,4-dimethyl-5-[(4-(dimethylamino)phenyl)azo]-1,2,4-triazolium chloride (CI11055; Basic Red No. 22), 2-hydroxy-1-[(2-methoxyphenyl)azo]-7-(trimethyl-ammonio)naphthalene chloride (CI12245; Basic Red No. 76), 2-[2-((2,4-dimethoxyphenyl)amino)ethenyl]-1,3,3-trimethyl-3H-indol-1-ium chloride (CI48055; Basic Yellow No. 11), 3-methyl-1-phenyl-4-[(3-(trimethylammonio)phenyl)azo]pyrazol-5-one chloride (CI12719; Basic Yellow No. 57), bis[4-(diethylamino)phenyl]phenylcarbenium hydrogensulphate (1:1) (CI42040; Basic Green No. 1), 1-[di(2-hydroxyethyl)amino]-3-methyl-4-[(4-nitrophenyl)azo]benzene (CI11210, Disperse Red No. 17), 4-[(4-aminophenyl)azo]-1-[di(2-hydroxyethyl)amino]-3-methylbenzene (HC Yellow No. 7), 2,6-diamino-3-[(pyridin-3-yl)azo]-pyridine, 6-hydroxy-5-[(4-sulphophenyl)azo]-2-naphthalenesulphonic acid disodium salt (CI15985; Food Yellow No. 3; FD&C Yellow No. 6), 2,4-dinitro-1-naphthol-7-sulphonic acid disodium salt (CI10316; Acid Yellow No. 1; Food Yellow No. 1), 2-(indane-1,3-dion-2-yl)quinoline-x,x-sulphonic acid (mixture of mono- and disulphonic acid) (CI47005; D&C Yellow No. 10; Food Yellow No. 13, Acid Yellow No. 3), 5-hydroxy-1-(4-sulphophenyl)-4-[(4-sulphophenyl)azo]pyrazole-3-carboxylic acid trisodium salt (CI19140; Food Yellow No. 4; Acid Yellow No. 23), 9-(2-carboxyphenyl)-6-hydroxy-3H-xanthen-3-one (CI45350; Acid Yellow No. 73; D&C Yellow No. 8), 5-[(2,4-dinitrophenyl)amino]-2-phenylaminobenzene-sulphonic acid sodium salt (CI10385; Acid Orange No. 3), 4-[(2,4-dihydroxyphenyl)azo]benzenesulphonic acid monosodium salt (CI14270; Acid Orange No. 6), 4-[(2-hydroxynaphth-1-yl)azo]-benzenesulphonic acid sodium salt (CI15510; Acid Orange No. 7), 4-[(2,4-dihydroxy-3-[(2,4-dimethylphenyl)azo]phenyl)azo]-benzenesulphonic acid sodium salt (CI20170; Acid Orange No. 24), 4-hydroxy-3-[(4-sulphonaphth-1-yl)azo]-1-naphthalenesulphonic acid disodium salt (CI14720; Acid Red No. 14), 6-hydroxy-5-[(4-sulphonaphth-1-yl)azo]-2,4-naphthalenedisulphonic acid trisodium salt (CI16255; Ponceau 4R; Acid Red No. 18), 3-hydroxy-4-[(4-sulphonaphth-1-yl)azo]-2,7-naphthalenedisulphonic acid trisodium salt (CI16185; Acid Red No. 27), 8-amino-1-hydroxy-2-(phenylazo)-3,6-naphthalenedisulphonic acid disodium salt (CI17200; Acid Red No. 33), 5-(acetylamino)-4-hydroxy-3-[(2-methylphenyl)azo]-2,7-naphthalenedisulphonic acid disodium salt (CI18065; Acid Red No. 35), 2-(3-hydroxy-2,4,5,7-tetraiododibenzopyran-6-on-9-yl)benzoic acid disodium salt (CI45430; Acid Red No. 51), N-[6-(diethylamino)-9-(2,4-disulphophenyl)-3H-xanthen-3-ylidene]-N-ethyl-ethaneammonium hydroxide, internal salt, sodium salt (CI45100; Acid Red No. 52), 8-[(4-(phenylazo)phenyl)azo]-7-naphthol-1,3-disulphonic acid disodium salt (CI27290; Acid Red No. 73), 2',4',5',7'-tetrabromo-3',6'-dihydroxyspiro[isobenzofuran-1(3H),9'-[9H]xanthen]-3-one disodium salt (CI45380; Acid Red No. 87), 2',4',5',7'-tetrabromo-4,5,6,7-tetrachloro-3',6'-dihydroxyspiro[isobenzofuran-1(3H),9'-[9H]xanthen]-3-one disodium salt (CI45410; Acid Red No. 92), 3',6'-dihydroxy-4',5'-diiodospiro[isobenzo-furan-1 (3H),9'(9H)-xanthen]-3-one disodium salt (CI45425; Acid Red No. 95), (2-sulphophenyl)di[4-(ethyl((4-sulphophenyl)methyl)amino)phenyl]-carbenium disodium salt, betaine (CI42090; Acid Blue No. 9; FD&C Blue No. 1), 1,4-bis[(2-sulpho-4-methylphenyl)amino]-9,10-anthraquinone disodium salt (CI61570; Acid Green No. 25), bis[4-(dimethylamino)phenyl]-(3,7-disulpho-2-hydroxynaphth-1-yl)carbenium internal salt, monosodium salt (CI44090; Food Green No. 4; Acid Green No. 50), bis[4-(diethylamino)phenyl](2,4-disulphophenyl)carbenium internal salt, sodium salt (2:1) (CI42045; Food Blue No. 3; Acid Blue No. 1), bis[4-(diethylamino)phenyl](5-hydroxy-2,4-disulphophenyl)carbenium internal salt, calcium salt (2:1) (CI42051; Acid Blue No. 3), 1-amino-4-(cyclohexylamino)-9,10-anthraquinone-2-sulphonic acid sodium salt (CI62045; Acid Blue No. 62), 2-(1,3-dihydro-3-oxo-5-sulpho-2H-indol-2-ylidene)-2,3-dihydro-3-oxo-1H-indole-5-sulphonic acid disodium salt (CI73015; Acid Blue No. 74), 9-(2-carboxyphenyl)-3-[(2-methylphenyl)-amino]-6-[(2-methyl-4-sulphophenyl)amino]xanthylium internal salt, monosodium salt (CI45190; Acid Violet No. 9), 1-hydroxy-4-[(4-methyl-2-sulphophenyl)amino]-9,10-anthraquinone sodium salt (CI60730; D&C Violet No. 2; Acid Violet No. 43), 3,3'- [sulphonylbis(2-nitro-4,1-phenylene)imino]bis[6-(phenylamino]-benzenesulfonic acid disodium salt (CI10410; Acid Brown No. 13), 5-amino-4-hydroxy-6-[(4-nitrophenyl)azo]-3-(phenylazo)-2,7-naphthalenedisulphonic acid disodium salt (CI20470; Acid Black No. 1), 3-hydroxy-4-[(2-hydroxynaphth-1-yl)azo]-7-nitro-1-naphthalenesulphonic acid-chromium complex (3:2) (CI15711; Acid Black No. 52), 3-[(2,4-dimethyl-5-sulphophenyl)azo]-4-hydroxy-1-naphthalenesulphonic acid disodium salt (CI14700; Food Red No. 1; FD&C Red No. 4), 4-(acetylamino)-5-hydroxy-6-[(7-sulpho-4-[(4-sulphophenyl)azo]naphth-1-yl)azo]-1,7-naphthalenedisulphonic acid tetrasodium salt (CI28440; Food Black No. 1) and 3-hydroxy-4-(3-methyl-5-oxo-1-phenyl-4,5-dihydro-1H-pyrazol-4-ylazo)naphthalene-1-sulphonic acid sodium salt chromium complex (Acid Red No. 195), on their own or in combination with one another.

The total amount of the direct dyes in the agent according to the invention is about 0.01 to 7 per cent by weight, preferably about 0.2 to 4 per cent by weight.

Further known and customary dyes for colouring hair which may be present in the colorant according to the invention are described, inter alia, in E. Sagarin, "Cosmetics, Science and Technology", Interscience Publishers Inc., New York (1957), pages 503 ff. and H. Janistyn, "Handbuch der Kosmetika und Riechstoffe" [Handbook of Cosmetics and Fragrances], volume 3 (1973), pages 388 ff. and K. Schrader "Grundlagen und Rezepturen der Kosmetika" [Fundamentals and Formulations of Cosmetics], 2nd edition (1989), pages 782-815.

Although oxidation colorants are preferred, it is of course likewise possible for the colorant according to the invention to be in the form of a non-oxidative colorant based on the abovementioned direct dyes.

Moreover, antioxidants, such as, for example, ascorbic acid, thioglycolic acid or sodium sulphite, and complexing agents for heavy metals, for example ethylenediaminotetraacetate or nitriloacetic acid, may be present in the agent according to the invention in an amount of up to about 0.5 per cent by weight. Perfume oils may be present in the colour carrier mass according to the invention in an amount of up to about 1 per cent by weight. The hair colorant described above can of course optionally comprise further additives customary for hair colorants, such as, for example, preservatives; complexing agents; solvents, such as water, lower aliphatic alcohols, for example aliphatic alcohols with 1 to 4 carbon atoms, such as ethanol, propanol and isopropanol, or glycols, such as glycerol and 1,2-propylene glycol; wetting agents or emulsifiers from the classes of anionic, cationic, amphoteric or nonionogenic surface-active substances; also softeners; vaseline; silicone oils, paraffin oil and fatty acids, and in addition care substances, such as cationic resins, lanolin derivatives, cholesterol, vitamins, pantothenic acid and betaine. The constituents mentioned are used in the amounts customary for such purposes, for example the wetting agents and emulsifiers in concentrations of from 0.1 to 30 per cent by weight and the care substances in a concentration of from 0.1 to 5.0 per cent by weight. Of particular advantage here is the addition of nonionic and/or anionic surfactants or emulsifiers, such as, for example, fatty alcohol sulphates, in particular lauryl sulphate or sodium cocoyl sulphate, oxyethylated fatty alcohol sulphates, in particular sodium lauryl ether sulphates having 2 to 4 ethylene oxide units in the molecule, oxyethylated fatty acid esters, oxyethylated nonylphenols, oxyethylated fatty alcohols, alkylbenzene-sulphonates or fatty acid alkanolamides, in a total amount of from about 0.1 to 30 per cent by weight, preferably 0.2 to 15 per cent by weight.

The pH of the colorant according to the invention for non-oxidative colorants based on direct dyes is in the range from about 5 to 10, preferably 6 to 9, while for oxidative colorants based on oxidation dye precursors the pH is in the range from about 6 to 12, preferably 9 to 11, the pH of the ready-to-use oxidation hair colorant (i.e. of the mixture of the hair colorant according to the invention with the oxidizing agent) is about 5.5 to 10, preferably 6 to 9.

Depending on the composition and desired pH of the colorant, the pH is adjusted preferably using ammonia or organic amines, such as, for example, glucamines, aminomethylpropanol, monoethanolamine or triethanolamine, inorganic bases, for example sodium hydroxide, potassium hydroxide, sodium carbonate or calcium hydroxide, or organic or inorganic acids, such as; for example, lactic acid, citric acid, acetic acid or phosphoric acid.

The agent according to the invention is preferably formulated in the form of an aqueous or aqueous-alcoholic preparation, for example as a thickened solution, emulsion, cream or gel. For the use for the oxidative colouring, the above-described colorant is mixed directly prior to use with an oxidizing agent, and an amount of the ready-to-use preparation sufficient for the coloration, generally about 60 to 200 grams, is applied to the fibres.

The agent according to the invention can also be formulated in the form of a multicomponent agent in which the dyes are present as a separate component which are mixed directly prior to use with an oxidizing agent and a dye-free preparation comprising the cellulose ether according to the invention and the remaining components. It is likewise possible to incorporate the cellulose ether according to the invention into the oxidizing agent, the colorant optionally likewise comprising the cellulose ether according to the invention, and the dyes may be present in this agent or else be separate and only mixed in directly prior to use. The cellulose ether according to the invention can also be added in separate form as a so-called added thickener, where the colorant and the oxidizing agent may optionally already be mixed together.

If the colorant according to the invention comprises no oxidation dye precursors or comprises oxidation dye precursors which are readily oxidizable with atmospheric oxygen, it can be applied directly to the keratin fibres without prior mixing with an oxidizing agent.

Suitable oxidizing agents for developing the coloration are primarily hydrogen peroxide or its addition compounds onto urea, melamine or sodium borate in the form of a 1 to 12 per cent strength, preferably 1.5 to 6 per cent strength, aqueous solution. The mixing ratio of colorant to oxidizing agent is dependent on the concentration of the oxidizing agent and is generally about 5:1 to 1:2, preferably 1:1, where the content of oxidizing agent in the ready-to-use preparation is preferably about 0.5 to 8 per cent by weight, in particular 1 to 4 per cent by weight.

The ready-to-use colorant is left to act on the keratin fibres (for example human hair) at 15 to 50 °C for about 10 to 45 minutes, preferably about 15 to 30 minutes, then the fibres are rinsed with water and dried. If required, this rinsing is followed by washing with a shampoo and possibly after-rinsing with a weak organic acid, such as, for example, tartaric acid. The keratin fibres are then dried.

If required, the viscosity of the oxidation colorant according to the invention can also be subsequently directly increased further after mixing with the oxidizing agent by adding the cationic cellulose ethers according to the invention, as a result of which simpler and more cost-effective base formulations are possible. It is likewise possible to add the cationic cellulose ether according to the invention to the colorant only directly prior to use (before, after or while mixing with the oxidizing agent) and thus establish the desired viscosity.

The colorant according to the invention satisfies the requirements with regard to the adhesion properties, the care properties, the application behaviour and the viscosity adjustment in an excellent manner and is noticeably easier to apply than comparable compositions. In addition, the colorants according to the invention have a uniform consistency and a cosmetic elegance. In particular, the very good viscosity and the excellent stability of the agent according to the invention, and its exceptional adhesion to the hair should be emphasized. In addition, the use of the agent according to the invention permits a variation in the weight ratio of colorant to oxidizing agent over a wide range (for example 1:1 to 1:3) without noteworthy impairment of the viscosity and adhesion properties of the ready-to-use oxidation colorant.

The examples below are intended to illustrate the subject-matter of the invention in more detail without limiting it thereto.

### Examples

### Example 1: Multicomponent oxidation hair colorant

**Component (A): Cream-like colour carrier mass (dye-free)**

| | |
|---|---|
| 7.0000 g | cetylstearyl alcohol |
| 3.0000 g | glycol distearate |
| 3.0000 g | polyethylene glycol(25) cetylstearyl ether |
| 9.0000 g | coconut fatty acid monoethanolamide |
| 3.0000 g | polyethylene glycol(7) glyceryl monococoate |
| 4.0000 g | lauryl ether sulphate |
| 0.2000 g | Dow Corning Softcat® SL-30 (cationic cellulose ether according to the invention) |
| 0.1000 g | ethylenediamineacetic acid |
| 0.2000 g | ascorbic acid |
| 0.4000 g | sodium sulphite |
| 0.2500 g | perfume |
| 9.5000 g | ammonia, 25% strength aqueous solution |
| ad 100.0000 g | water, demineralized |

**Component (B): Dye powder or dye granules**

| | |
|---|---|
| 7.249 g | 4-aminophenol |
| 2.661 g | 1-naphthol |
| 0.072 g | resorcinol |
| 0.018 g | 2-amino-6-chloro-4-nitrophenol |

50 g of the above component (A) are mixed directly prior to use with 10 g of component (B) and 50 g of a 6% strength aqueous hydrogen peroxide solution. A homogeneous, cosmetically elegant, optimally thickened colouring preparation is obtained. The mixture obtained in this way is then applied to mid-blonde natural hair. After a contact time of 30 minutes at 40 °C, the hair is rinsed with water and dried. The hair is given a brown-red, lustrous coloration.

### Example 2: Oxidation hair colorant, liquid

| | |
|---|---|
| 0.3000 g | Dow Corning Softcat® SL-60 (cationic cellulose ether according to the invention) |
| 5.0000 g | 2-octyl-1-dodecanol |
| 15.0000 g | oleic acid |
| 10.0000 g | sodium lauryl alcohol diglycol ether sulphate (28% strength aqueous solution) |
| 1.3620 g | 4-aminophenol |
| 0.5000 g | 1-naphthol |
| 0.0136 g | resorcinol |
| 0.0034 g | 2-amino-6-chloro-4-nitrophenol |
| 12.0000 g | ammonia, 25% strength aqueous solution |
| 1.0000 g | ethylenediaminotetraacetate disodium salt |
| 1.0000 g | ascorbic acid |
| 15.0000 g | isopropanol |
| ad 100.0000 g | water |

50 g of the above hair colorant are mixed directly prior to use with 50 g of a 6 per cent strength aqueous hydrogen peroxide solution. A homogeneous, cosmetically elegant, optimally thickened colouring preparation is obtained. The mixture obtained in this way is then applied to blonde natural hair. After a contact time of 30 minutes at 40 °C, the hair is rinsed with water and dried. The hair is given a lustrous, copper-red coloration.

### Example 3: Gel-like oxidation hair colorant for lightening

**Component (A): Liquid colour carrier mass**

| | |
|---|---|
| 0.25 g | Dow Corning Softcat® SL-100 (cationic cellulose ether according to the invention) |
| 10.00 g | lauryl alcohol |
| 6.00 g | nonylphenol, oxyethylated with 4 mol ethylene oxide |
| 6.00 g | oleic acid |
| 0.50 g | p-phenylenediamine |
| 0.07 g | resorcinol |
| 5.00 g | sodium lauryl alcohol diglycol ether sulphate, 28% strength aqueous solution |
| 1.00 g | ethylenediamineacetic acid disodium salt |
| 18.00 g | ammonia, 25% strength aqueous solution |
| 8.00 g | ethanol |
| ad 100.00 g | water |

**Component (B): Hydrogen peroxide emulsion**

| | |
|---|---|
| 10.0 g | cetylstearyl alcohol |
| 1.5 g | cholesterol |
| 4.0 g | sodium lauryl alcohol diglycol ether sulphate, 28% strength aqueous solution |
| 35.0 g | hydrogen peroxide, 35% strength aqueous solution |
| 0.3 g | perfume |
| ad 100.0 g | water |

Prior to use, 40 g of the liquid colour carrier mass (A) are mixed with 80 g of the hydrogen peroxide emulsion (B), corresponding to a mixing ratio of (A):(B) of 1:2, and 120 g of this mixture is applied to grey human hair. After a contact time of 20 minutes at room temperature, the hair is rinsed with water and dried. The hair treated in this way is coloured pale brown evenly from the hair roots to the hair ends. The agent according to the invention can be applied easily and does not run off from the hair.

### Example 4: Oxidation hair colorant, cream-like

| | |
|---|---|
| 0.15 g | Dow Corning Softcat® SL-30 (cationic cellulose ether according to the invention) |
| 3.00 g | oleyl alcohol |
| 15.00 g | cetyl alcohol |
| 3.50 g | sodium lauryl alcohol diglycol ether sulphate (28% strength aqueous solution) |
| 3.00 g | monoethanolamine |
| 1.30 g | 1-methyl-2,5-diaminobenzene |
| 1.00 g | beeswax |
| 0.65 g | resorcinol |
| 0.50 g | keratin hydrolysate |
| 0.50 g | silk protein hydrolysate |
| 0.50 g | 2-amino-6-chloro-4-nitrophenol |
| 0.30 g | ascorbic acid |
| ad 100.00 g | water |

50 g of the above hair colorant are mixed directly prior to use with 50 g of a 12% strength aqueous hydrogen peroxide solution. The resulting mixture is then applied to blonde natural hair. After a contact time of 30 minutes at 40 °C, the hair is rinsed with water and dried. A uniform, rich brown shade is obtained.

### Example 5: Nonoxidative hair tint

| | |
|---|---|
| 2.0 g | Dow Corning Softcat® SL-60 (cationic cellulose ether according to the invention) |
| 6.0 g | lauryl alcohol |
| 5.0 g | sodium lauryl sulphate |
| 3.0 g | cetylstearyl alcohol and sodium lauryl sulphate (Lanette W) |
| 1.5 g | 2-amino-6-chloro-4-nitrophenol |
| 1.0 g | monoethanolamine |
| 1.0 g | beeswax |
| 0.5 g | keratin hydrolysate |
| 0.3 g | silk protein hydrolysate |
| 0.2 g | glycine |
| ad 100.0 g | water |

A slightly gel-like colouring mass is obtained which, on account of its excellent viscosity properties, can be applied easily and uniformly, and adheres very readily to the hair. After a contact time of 20 minutes at 20 to 25 °C, the hair is rinsed with lukewarm water, styled and dried. The hair treated in this way has a uniform, richly lustrous gold-orange coloration.

### Example 6: Oxidation hair colorant, cream-like

**Component (A): Colour carrier mass**

| | |
|---|---|
| 2.0 g | Dow Corning Softcat® SL-30 (cationic cellulose ether according to the invention) |
| 8.0 g | 2-octyl-1-dodecanol |
| 3.0 g | sodium lauryl alcohol diglycol ether sulphate, 28% strength aqueous solution |
| 2.8 g | 2,5-diaminotoluene sulphate |
| 1.0 g | resorcinol |
| 0.4 g | m-aminophenol |
| 0.2 g | 2-amino-4-(2'-hydroxyethylamino)anisol sulphate |
| 0.3 g | ascorbic acid |
| 0.1 g | ethylenediaminetetraacetic acid |
| 12.2 g | ammonia, 25% strength aqueous solution |
| 2.0 g | ethanol |
| ad 100.0 g | water |

**Component (B): Hydrogen peroxide emulsion**

| | |
|---|---|
| 10.0 g | cetylstearyl alcohol |
| 1.5 g | cholesterol |
| 4.0 g | sodium lauryl alcohol diglycol ether sulphate, 28% strength aqueous solution |
| 17.0 g | hydrogen peroxide, 35% strength aqueous solution |
| 0.3 g | perfume |
| ad 100.0 g | water |

Prior to use, 40 g of the liquid colour carrier mass (A) are mixed with 80 g of the hydrogen peroxide emulsion (B), corresponding to a mixing ratio of (A):(B) of 1:2, and 120 g of this mixture are applied to grey human hair. After a contact time of 20 minutes at room temperature, the hair is rinsed with water and dried. The hair treated in this way has taken on an even, dark-brown shade. The agent according to the invention adheres very readily to the hair without running off.

### Example 7: Multicomponent oxidation hair colorant

**Component (A): Colour carrier mass**

| | |
|---|---|
| 2.0 g | Dow Corning Softcat® SL-30 (cationic cellulose ether according to the invention) |
| 8.0 g | 2-octyl-1-dodecanol |
| 9.0 g | cetearyl alcohol |
| 3.0 g | sodium lauryl alcohol diglycol ether sulphate, 28% strength aqueous solution |
| 0.3 g | ascorbic acid |
| 0.1 g | ethylenediaminetetraacetic acid |
| 12.2 g | ammonia, 25% strength aqueous solution |
| 2.0 g | ethanol |
| ad 100.0 g | water |

The dyes/dye mixtures listed in Table 1 below are added in pulverized or granulated form.

**Table 1:**

| **Dyes/colour shade** (amounts in grams) | yellow | gold | orange | red | violet | pink | blue | brown | green |
|---|---|---|---|---|---|---|---|---|---|
| p-Tolylenediamine sulphate | | | | | | | | | 4.95 |
| 2,4-Diaminophenoxyethanol*HCl | | | | | | | 3.86 | | |
| p-Phenylenediamine | | | | | | | | 2.40 | |
| p-Aminophenol | | | 1.50 | | | | | | |
| Resorcinol | | | | | | | | 1.80 | |
| m-Aminophenol | | | | | | | | 0.64 | |
| 4-Amino-2-hydroxytoluene | | | 2.05 | 1.12 | 1.17 | | | | |
| 1-Naphthol | | | | | | 1.73 | | | |
| 6-Amino-3-methyl-phenol | 1.50 | | | | | | | | |
| Hydroxyethyl-3,4-methylenedioxyaniline*HCl | | | | | | | | | 4.90 |
| N,N-bis(2-Hydroxyethyl)-p-phenylenediamine sulphate | | | | | 2.80 | | 4.71 | | |
| 1-Hydroxyethyl-4,5-diaminopyrazole sulphate | | | | 2.18 | | 2.88 | | | |
| 2-Amino-6-chloro-4-nitrophenol | | 2.00 | | | | | | | |
| N(2-Hydroxyethyl)-2-nitro-4-trifluoromethylaniline | | | | | | | | | 3.00 |

The base shades can be mixed together as desired in order to achieve a broad spectrum of nuances.

**Component (B): Hydrogen peroxide emulsion**

| | |
|---|---|
| 10.0 g | cetylstearyl alcohol |
| 1.5 g | cholesterol |
| 4.0 g | sodium lauryl alcohol diglycol ether sulphate, 28% strength aqueous solution |
| 35.0 g | hydrogen peroxide, 35% strength aqueous solution |
| 0.3 g | perfume |
| ad 100.0 g | water |

Prior to use, 40 g of the creamy colour carrier mass (A) are mixed with 80 g of the hydrogen peroxide emulsion (B), corresponding to a mixing ratio of (A):(B) of 1:2, the dyes/dye mixtures listed in Table 1 are added depending on the desired colour shade, the entire mixture is stirred and 120 g of this mixture are applied to grey human hair. After a contact time of 20 minutes at room temperature, the hair is rinsed with water and dried. The hair treated in this way has taken on a shade corresponding to the base shade mixture. The agent according to the invention adheres very readily to the hair without running off.

### Example 8: Oxidation hair colorant, cellulose ether in the peroxide component

**Component (A): Colour carrier mass**

| | |
|---|---|
| 8.0 g | 2-octyl-1-dodecanol |
| 3.0 g | sodium lauryl alcohol diglycol ether sulphate, 28% strength aqueous solution |
| 2.8 g | 2,5-diaminotoluene sulphate |
| 1.0 g | resorcinol |
| 0.4 g | m-aminophenol |
| 0.2 g | 2-amino-4-(2'-hydroxyethylamino)anisol sulphate |
| 0.3 g | ascorbic acid |
| 0.1 g | ethylenediaminetetraacetic acid |
| 12.2 g | ammonia, 25% strength aqueous solution |
| 2.0 g | ethanol |
| ad 100.0 g | water |

**Component (B): Hydrogen peroxide emulsion**

| | |
|---|---|
| 1.0 g | Dow Corning Softcat® SL-30 (cationic cellulose ether according to the invention) |
| 10.0 g | cetylstearyl alcohol |
| 1.5 g | cholesterol |
| 4.0 g | sodium lauryl alcohol diglycol ether sulphate, 28% strength aqueous solution |
| 25.7 g | hydrogen peroxide, 35% strength aqueous solution |
| 0.3 g | perfume |
| ad 100.0 g | water |

Prior to use, 40 g of the liquid colour carrier mass (A) are mixed with 80 g of the hydrogen peroxide emulsion (B), corresponding to a mixing ratio of (A):(B) of 1:2, and 120 g of this mixture are applied to grey human hair. After a contact time of 20 minutes at room temperature, the hair is rinsed with water and dried. The hair treated in this way has taken on an even, dark-brown shade. The agent according to the invention adheres very readily to the hair without running off.

### Example 9: Cationic cellulose ether as subsequent colour thickener

**Component (A): Colour thickener with cellulose ether**

| | |
|---|---|
| 5.0 g | Dow Corning Softcat® SL-30 (cationic cellulose ether according to the invention) |
| 0.3 g | perfume |
| 10.0 g | ethanol |
| ad 100.0 g | water |

**Component (B): Liquid colorant**

| | |
|---|---|
| 10.00 g | lauryl alcohol |
| 6.00 g | nonylphenol, oxyethylated with 4 mol of ethylene oxide |
| 6.00 g | oleic acid |
| 0.50 g | p-phenylenediamine |
| 0.07 g | resorcinol |
| 5.00 g | sodium lauryl alcohol diglycol ether sulphate, 28% strength aqueous solution |
| 1.00 g | ethylenediaminetetraacetic acid disodium salt |
| 18.00 g | ammonia, 25% strength aqueous solution |
| 8.00 g | ethanol |
| ad 100.00 g | water |

**Component (C): hydrogen peroxide emulsion**

| | |
|---|---|
| 10.0 g | cetylstearyl alcohol |
| 1.5 g | cholesterol |
| 4.0 g | sodium lauryl alcohol diglycol ether sulphate, 28% strength aqueous solution |
| 35.0 g | hydrogen peroxide, 35% strength aqueous solution |
| 0.3 g | perfume |
| ad 100.0 g | water |

Prior to use, 40 g of the liquid colour carrier mass (A) are mixed with 40 g of the hydrogen peroxide emulsion (B), corresponding to a mixing ratio of (A):(B) of 1:1, the colour thickener (A) is added until the desired viscosity is achieved (about 1 to 3 g) and this mixture is applied to grey human hair. After a contact time of 20 minutes at room temperature, the hair is rinsed with water and dried. The hair treated in this way has taken on an even, dark-brown shade. The agent according to the invention adheres very readily to the hair without running off.

Unless stated otherwise, all of the per centages given in the present application are per centages by weight.

## Claims

1. Agent for colouring keratin fibres based on oxidation dye precursors and/or direct dyes, **characterized in that** it comprises, in a suitable cosmetic carrier, at least one cellulose ether with 4,000 to 10,000 anhydroglucose units, which is substituted by (a) per mole of anhydroglucose unit on average 0.0003 to 0.08 mol of a nonionic or cationic hydrophobic substituent containing an alkyl group or arylalkyl group having 8 to 24 carbon atoms and (b) a cationic substituent of the formula (I),
[R¹R²R³R⁴N⁺] (A^{z-})_{1/z} (I)
where
**R1, R2** and **R3,** independently of one another, are a methyl or ethyl group,
**R4** is a -CH2-CH2 group or a -CH2-CHOH-CH2 group,
**A^{z-}** is an anion and z is 1, 2 or 3.

2. Agent according to Claim 1, **characterized in that** the hydrophobic substituent (a) is a compound of the formula (II),
[R⁵R⁶R⁷R⁸N⁺] (A^{z-})_{1/z} (II)
where R5 and R6, independently of one another, are a methyl group or ethyl group,
**R7** is a -CH2-CH2 group or a -CH2-CHOH-CH2 group,
**R8** is an alkyl or arylalkyl group having 8 to 24 carbon atoms
**A^{z-}** is an anion and **z** is 1, 2 or 3.

3. Agent according to Claim 2, **characterized in that** the radical **R5** is methyl.

4. Agent according to Claim 2 or 3, **characterized in that** the radicals **R5** and R6 are methyl.

5. Agent according to one of Claims 2 to 4, **characterized in that** the radical R7 is a -CH2-CHOH-CH2 group.

6. Agent according to one of Claims 2 to 5, **characterized in that** the radical R8 is an alkyl group having 8 to 24 carbon atoms, a glycidyl ether, an alpha-olefin epoxide or an alkyl halide.

7. Agent according to one of Claims 1 to 6, **characterized in that**, in the formula (I), **R1** is methyl and **R4** is -CH2-CHOH-CH2 and the degree of substitution of the cationic substituent (b) is on average 0.002 to 0.9 mol.

8. Agent according to one of Claims 1 to 7, **characterized in that** the hydrophobic substituent (a) is a
[-O-(CH₂CH₂O)ₓ-(CH₂CHOHCH-N(CH₃)₂(C₁₂H₂₅)⁺Cl⁻)_{y}-H] group and the cationic substituent (b) is a [-O-(CH₂CH₂O)ₓ-(CH₂CHOHCH-N(CH₃)₃⁺Cl⁻)_{y}-H] group.

9. Agent according to one of Claims 1 to 8, **characterized in that** the cationic cellulose ether is present in an amount of from 0.01 to 20 per cent by weight.

10. Agent according to one of Claims 1 to 9, **characterized in that** it is a hair colorant.

11. Agent according to one of Claims 1 to 10, **characterized in that** it is mixed with an oxidizing agent prior to use.

12. Ready-to-use agent for colouring hair which is prepared directly prior to use by mixing a colour carrier mass comprising oxidation dye precursors and/or direct dyes, an oxidizing agent and a preparation comprising a cellulose ether with 4,000 to 10,000 anhydroglucose units which is substituted by (a) per mole of anhydroglucose unit on average 0.0003 to 0.08 mol of a nonionic or cationic hydrophobic substituent containing an alkyl group or arylalkyl group having 8 to 24 carbon atoms and (b) a cationic substituent of the formula (I),
[R¹R²R³R⁴N⁺] (A^{z-})_{1/z} (I)
where **R1, R2** and **R3,** independently of one another, are a methyl or ethyl group,
R4 is a -CH2-CH2 group or a -CH2-CHOH-CH2 group,
**A^{z-}** is an anion and z is 1, 2 or 3,
where the 3 components are mixed together in any order.
